# EUROPEAN PATENT SPECIFICATION

(11) **EP 0 806 483 B1**
(45) Date of publication and mention of the grant of the patent: **28.01.2004**
(21) Application number: 96939308.1
(22) Date of filing: 27.11.1996
(51) Int. Cl.: C12Q 1/68, C12N 15/11, C07H 21/04

(54) **DETECTION OF BACTERIUM BELONGING TO THE GENUS PECTINATUS**
NACHWEIS EINER BAKTERIE DER PECTINATUS GATTUNG
PROCEDE DE DETECTION D'UNE BACTERIE APPARTENANT AU GENRE PECTINATUS

(30) Priority: 28.11.1995 JP 33117295; 28.11.1995 JP 33117395
(43) Date of publication of application: 12.11.1997
(73) Proprietor: ASAHI BREWERIES, LTD., Tokyo 104 (JP)
(72) Inventor: SAKAMOTO, Kanta, 2-13-1, Oumori Kita Outa-ku Tokyo 143 (JP)
(74) Representative: Sheard, Andrew Gregory
(86) International application number: PCT/JP1996/003464
(87) International publication number: WO 1997/020071

(56) References cited:
- WO-A-94/10204
- WO-A-95/07289
- JOURNAL OF INDUSTRIAL MICROBIOLOGY, Vol. 15, Aug. 1995, L.M. DOYLE et al., "Sequence of the Gene Encoding the 16S rRNA of the Beer Spoilage Organism Megasphaera Cerevisiae", pages 67-70.
- GENETICS, Vol. 132, 1992, THOMAS MALVAR et al., "The CCR4 Protein From Saccharomyces Cerevisiae Contains a Leucine-Rich Repeat Region Which is Required for Its Control of ADH2 Gene Expression", pages 951-962.
- SYSTEM. APPL. MICROBIOL., Vol. 15, 1992, NORBERT KLUGBAUER et al., "Subunit beta of Adenosine Triphosphate Synthase of Pectinatus Frisingensis and Lactobacillus Casei", pages 323-330.
- INTERNATIONAL JOURNAL OF SYSTEMATIC BACTERIOLOGY, Vol. 40(1), 1990, KARL HEINZ SCHLEIFER et al., "Taxonomic Study of Anaerobic, Gram-Negative, Rod-Shaped Bacteria from Breweries: Emended Description of Pectinatus Cerevisiiphillus and Description of Pectinatus Frisingensis Sp. Nov., Selenomonas Lacticifex Sp. Nov., Zymophilus Raffinosivorans Gen. Nov. and Zymophilus Paucivorans Sp. Nov.", pages 19-27.

## Description

The present invention relates to detection of beer-spoilage bacteria of the genus *Pectinatus.* More particularly, the present invention relates to oligonucleotides for detecting *Pectinatus cerevisiiphilus, Pectinatus frisingensis* and *Pectinatus* sp. DSM20764, respectively.

In known methods for testing micro-organisms in the field of beer production, for identifying the species, the bacteria must be separated via growth cultivation and segregation cultivation, a process that takes at least seven days. Then, the separated bacteria are propagated, and identification is conducted by many tests, such as observation of the form and Gram stain properties, a catalase test, and presence of sugar consumption. These troublesome tests require many hours and are expensive. In addition to these conventional identification methods, there is a known method for identifying species by a hybridization test, in which DNA is extracted from an isolated bacteria and the DNA is fixed on a membrane or other substrate, and detected using the DNA of a standard bacteria as a probe. However, this method takes several days, and it is difficult to obtain the necessary detection sensitivity.

Recently, a method for detecting living bacteria by an ATP luminescence method has been reported in J. Am. Soc. Brew. Chem.: 52(1) 19-23, 1994. Although this method can detect bacteria quickly, it is unable to identify the bacteria. Further, as disclosed in Japanese Patent Laid-open Publication Nos. 6-46811(1994) and 6-311894(1994), it is possible to identify lactic acid bacteria, for example, *Lactobacillus brevis, Lactobacillus plantarum* and *Lactobacillus coryniformis*,in a relatively early stage by using antibodies specific for each species. However, to apply the method, isolation of the bacteria is still required. Accordingly, it takes several more days, and it is difficult to obtain sufficient detection sensitivity and selectivity.

Quicker detection methods have been studied. Disclosed in Japanese Patent Laid-open Publication Nos. 5-15400(1993) and 6-141899(1994) and as reported in J. Am. Soc. Brew. Chem.: 52(3) 95-99, 1994, is a method for detecting lactic acid bacteria, wherein DNA of lactic acid bacteria is extracted and an oligonucleotide complementary for the DNA is functionally used as a primer.

The genus *Pectinatus* of an obligately anaerobic bacterium exerts a bad influence on the product beer. To detect the genus, a selective separation medium was examined (J. Am. Soc. Brew. Chem.: 52(3) 115-119, 1994), a group for determining a main antigen was identified by using an immunoblotting technique (FEMS. MICROBIOL. LETT.: 67(3) 307-311, 1990), and a method using a fluoroimmunoassay method was disclosed (J. Am. Soc. Brew. Chem.: 51(4) 158-163, 1993). However, satisfactory methods are not established in aspects of the detectable sensitivity, detection times and the like.

WO 95/07289 discloses a method for detecting the presence of microorganisms which cause the spoilage of beer by detecting 16S/23S ribosomal RNA of *Pediococcus* or *Lactobacillus* spp. Doyle *et al*, Journal of Industrial Microbiology, 1995, 15(2): 67-70 discloses that the 16S ribosomal RNA from *P. cerevisiiphilus* and *P. frisingensis* can be used to draw phylogenetic trees among beer-spoilage bacteria.

The present invention aims to develop a method that the genus Pectinatus is more quickly and sensitively detected and identified at the same time.

In one aspect, the present invention provides an oligonucleotide characterized in that the oligonucleotide targets a nucleotide sequence encoding a 16S ribosomal RNA gene of the genus *Pectinatus* to selectively detect *P. cerevisiiphilus* belonging to the genus *Pectinatus* in a test sample, wherein the oligonucleotide is complementary to the nucleotide sequence, and is one of the following: or one of the corresponding complementary sequences, (SEQ ID NOS. 6-9).

The corresponding complementary sequences are:

In another aspect, the present invention provides an oligonucleotide characterized in that the oligonucleotide targets a nucleotide sequence encoding a 16S ribosomal RNA gene of the genus *Pectinatus* to selectively detect *P. frisingensis* belonging to the genus *Pectinatus* in a test sample, wherein the oligonucleotide is complementary to the nucleotide sequence, and is one of the following: or one of the corresponding complementary sequences, (SEQ ID NOS. 14-17).

The corresponding complementary sequences are:

In another aspect, the present invention provides an oligonucleotide characterised in that the oligonucleotide targets a nucleotide sequence encoding a 16S ribosomal RNA gene of the genus *Pectinatus* to selectively detect *Pectinatus* sp. DSM20764 belonging to the genus *Pectinatus* in a test sample, wherein the oligonucleotide is complementary to the nucleotide sequence, and is one of the following:

The corresponding complementary sequences are:

The whole base sequences of the gene encoding the 16S ribosomal RNA gene of *Pectinatus* sp. DSM20764 is shown in SEQ ID NO.1. *Pectinatus* sp. DSM20764 is obtainable from DSM-Deutche Sammlung von Mikroorganismen und Zellkulturen GmbH.

Techniques for gene amplification are known, and include the Polymerase Chain Reaction method (abbreviated as an PCR method hereinafter: Science 230, 1350, 1985). This reaction aims to amplify a special gene sequence. This method is applied in the field of genetic study, lately in the medical field for rapid determination of viruses, and in the food field for rapid detection of bacteria, because it shows rapid and high sensitivity and high specificity. Using the PCR method, even though a small amount of target gene is in a sample, the target sequence between two primers is amplified several million times, and detectable copies are produced in large quantities. In the PCR method, it is necessary to release the component of nucleic acid. When the sample has no less than several molecules, the amplification reaction proceeds, so that PCR can be done only by simple pretreatment using a lytic enzyme or a surfactant. Accordingly, the PCR method is very useful in comparison with conventional bacteria detection methods.

The present invention has been developed to use these methods. In the present invention, the PCR method is conducted by using as a primer the oligonucleotide which is designed to hybridize specifically with 16S ribosomal genes of *Pectinatus cerevisiiphilus, Pectinatus frisingensis* or *Pectinatus* sp. DSM20764, so that the sequence to be recognized is detected. As a result whether the bacteria of *Pectinatus cerevisiiphilus, Pectinatus frisingensis* or *Pectinatus* sp. DSM20764 is present or not in the sample can be determined, rapidly and sensitively. The sample may be collected from beer or green beer in the brewing process or a sample from sewage in an anaerobic atmosphere. Further, the oligonucleotide used as a primer may be obtained by chemical synthesis or from natural products.

The base length of the target sequence on nucleotide sequence of the 16S ribosomal RNA gene of *Pectinatus cerevisiiphilus,* which is specified by two primers, is about 70 base pairs for the combination of the primers of SEQ ID NO.2 and SEQ ID NO.4, about 390 base pairs for the combination of the primers of SEQ ID NO.3 and SEQ ID NO.5, and 440 base pairs for the combination of the primers of SEQ ID NO.2 and SEQ ID NO.5. Since any combination of these primers is specific for *Pectinatus cerevisiiphilus*, it is possible to detect the species, and it is possible to identify more reliably by using two or more combinations of primers in parallel.

The base length of the target sequence on nucleotide sequence of the 16S ribosomal RNA gene of *Pectinatus frisingensis*, which is specified by two primers, is about 70 base pairs for the combination of the primers of SEQ ID NO. 10 and SEQ ID NO.12, about 390 base pairs for the combination of the primers of SEQ ID NO.11 and SEQ ID NO.13, and 440 base pairs for the combination of the primers of SEQ ID NO.10 and SEQ ID NO.13. Since any combination of these primers is specific for *Pectinatus frisingensis*, it is possible to detect the genus, and it is possible to identify more reliably by using two or more combinations of primers in parallel.

The base length of the target sequence on nucleotide sequence of the 16S ribosomal RNA gene of *Pectinatus* sp. DSM20764, which is specified by the primers of SEQ ID NO.18 and SEQ ID NO.19, is about 390 base pairs. Since the combination of these primers is specific for *Pectinatus* sp. DSM20764, it is possible to detect the genus.

A DNA polymerase thermostable at a temperature of 90°C or more may be used in the PCR. One cycle of temperature conditions in PCR is 90-98°C for a thermal denaturing reaction for changing a double stranded DNA into a single stranded DNA, 37-65°C for an annealing reaction to hybridize primers with DNA and 50-75°C for an elongation reaction to act a DNA polymerase. Several tens of cycles are conducted to amplify target sequences. After the PCR, the reaction products are separated by electrophoresis. The nucleic acids of the resulting products are stained by ethidium bromide and the like. When the base length of the amplified nucleotide sequence is equal to the base length of the above target sequence, the presence of *Pectinatus cerevisiiphilus, Pectinatus frisingensis* or *Pectinatus* sp. DSM20764 is decided. The amplified nucleotide sequence is also effectively detected by chromatography.

The following examples serve to illustrate the present invention. Reference is made to the accompanying drawings.

Fig. 1 shows the result of electrophoresis after the PCR of the samples of bacteria Nos. 2 and 5 in Table 1, using combinations of the primers of SEQ ID NOS.2-5.

Fig. 2 shows the result of electrophoresis after the PCR reaction of the samples of bacteria Nos. 2 and 5 in Table 1, using combinations of the primers of SEQ ID NOS.10-13.

Fig. 3 shows the result of electrophoresis after the PCR of the samples of bacteria Nos. 1-9 in Table 4, using the combination of the primers of SEQ ID NOS.18 and 19.

### (1) Detection method of Pectinatus cerevisiiphilus

### Preparation of samples

Ten strains of three kinds of *Pectinatus* as set out in Table 1 were used. To confirm the specificity of the primers for *Pectinatus cerevisiiphilus*, other bacteria and yeasts to be detected in the inspection of beer microorganisms, and the *E. coli* used often in laboratories, were used. After cultivating these strains at suitable cultures for amplification, the strains were collected by centrifugation. Then, the DNA from the strains were extracted in accordance with the description of SHIN-SEIKAGAKU-JIKKEN-KOZA 2, Nucleic acid I, Separation and Purification, page 20-21 (edited by Japan Biochemical Learned Society, Tokyo-Kagaku-Dojin) to obtain each one ml of DNA solution.

**Table 1**

| Bacteria No. | Bacteria type | Name of strain | Remarks |
|---|---|---|---|
| 1 | *Pectinatus cerevisiiphilus* | DSM20466 | |
| 2 | *Pectinatus cerevisiiphilus* | DSM20467 | type strain, ATCC29359 |
| 3 | *Pectinatus cerevisiiphilus* | DSM20762 | |
| 4 | *Pectinatus cerevisiiphilus* | DSM20763 | |
| 5 | *Pectinatus frisingensis* | DSM6036 | type strain, ATTC33332 |
| 6 | *Pectinatus frisingensis* | DSM20465 | |
| 7 | *Pectinatus frisingensis* | DSM20759 | |
| 8 | *Pectinatus frisingensis* | DSM20760 | |
| 9 | *Pectinatus frisingensis* | DSM20761 | |
| 10 | *Pectinatus* sp. | DSM20764 | |
| 11 | *Lactobacillus brevis* | JCM1059 | type strain |
| 12 | *Lactobacillus casei* | ATCC334 | type strain |
| 13 | *Lactobacillus coryniformis* | JCM1164 | type strain |
| 14 | *Lactobacillus lindneri* | DSM20690 | type strain |
| 15 | *Lactobacillus plantarum* | JCM1149 | type strain |
| 16 | *Lactococcus lactis* | JCM5805 | type strain |
| 17 | *Lactococcus lactis* | JCM6123 | type strain |
| 18 | *Pediococcus damnosus* | JCM5886 | type strain |
| 19 | *Megasphaera cerevisiae* | DSM20462 | type strain |
| 20 | *Megasphaera cerevisiae* | JCM6129 | |
| 21 | *Zymophilus raffinosivorans* | DSM20765 | type strain |
| 22 | *Zymophilus paincivorans* | DSM20756 | type strain |
| 23 | *Saccharomyces cerevisiae* | 1 | beer brewer's yeast |
| 24 | *Saccharomyces cerevisiae* | 2 | beer brewer's yeast |
| 25 | *Saccharomyces cerevisiae* | 3 | beer brewer's yeast |
| 26 | *Escherichia coli* | K-12 | |

### Synthesis of Primers

Referring to the base sequences of 16S ribosomal RNA gene of *Pectinatus* (KARL HEINZ SCHLEIFER, and HELGA SEIDEL-RUFER *et al*, Int. J. Syst. Bacteriol. 40(1): 19-27, 1990), the following four sequences were selected, and oligonucleotides having these sequences were chemically synthesized.

### PCR

To a sterilized tube of 0.5 ml, the above sample solution of 1 µl, sterilized water of 78.5 µl, 10 x buffer for reaction of 10µl (manufactured by Toyobo Co., Ltd., 10 x TAP), 6 µl of water solution of 25 mM magnesium chloride, one µl of 20 mM dNTPs, each one µl of 100 µM of primer SEQ ID NO.2 and 100 µM of primer SEQ ID NO.4 (or Primers SEQ ID NOS.3 and 5, or SEQ ID NOS.2 and 5), and 0.5 µl of 5U/ µl Taq DNA polymerase (manufactured by Toyobo Co., Ltd., TAP-101) were added, and 100 µl of reaction solution was prepared. PCR was repeated for 30 cycles, each cycle having the following temperature conditions:
thermal denaturing; 94°C for one minute,
annealing; 54°C for one minute and
elongation; 74°C for one minute.

Before the cycle reaction, the solution was reacted at a temperature of 98°C for one minute. At the end of the reaction, the solution was heated to 74°C for three minutes. The program tempcontrol system PC-800 manufactured by ASTEC company was used as a thermal control unit.

### Detection

Using 10 µl of reaction solution after the PCR, electrophoresis was conducted by 3.5% (w/v) agarose gel at 100V constant voltage for 30 minutes. At the same time, ø X174/HincII was electrophoresed as a molecular weight marker. After the electrophoresis, the gel was dyed in a ethidium bromide solution (about 0.5 µg/ml) for 15 minutes, the gel was observed under ultraviolet irradiation, and photographed. By the observation or photograph of the gel, the base length of the amplified products was determined from relative migration distance to the molecular weight marker.

### Result

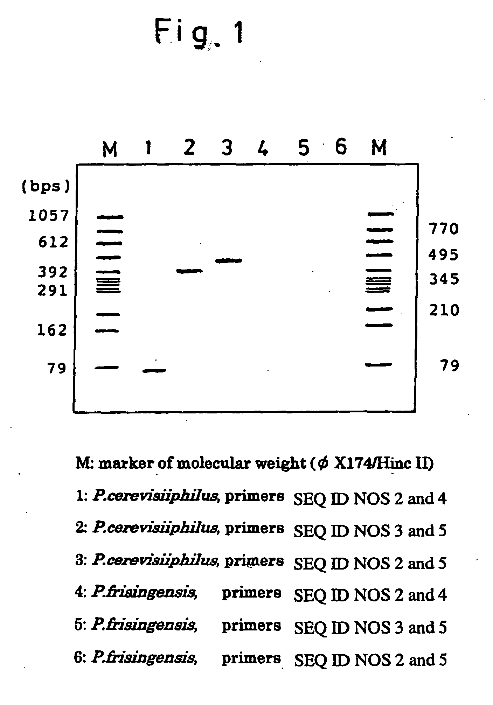

The result of the electrophoresis of the samples of genus numbers 2 and 5 in Table 1 after PCR is shown in Fig. 1.
The results of detected bands of all tested genus is shown in Table 2.

**Table 2**

| Genus No. | Combination of primers | | |
|---|---|---|---|
| | SEQ ID NOS. 2 & 4 | SEQ ID NOS.3 & 5 | SEQ ID NOS.2 & 5 |
| 1 | 0.07 | 0.39 | 0.44 |
| 2 | 0.07 | 0.39 | 0.44 |
| 3 | 0.07 | 0.39 | 0.44 |
| 4 | 0.07 | 0.39 | 0.44 |
| 5-26 | - | - | - |

In the table, figures show base length (kilo base pairs) of detected bands, and (-) shows no band.

From the result, when each oligonucleotide of SEQ ID NOS.2-5 was used as a primer of the PCR in the combination as shown in Table 2, a band was detected only in respect of *Pectinatus cerevisiiphilus,* and the band showed a base length equal to that of the target sequence. Therefore, it is shown that each oligonucleotide properly recognizes the sequence targeted by the 16S ribosomal RNA gene of *Pectinatus cerevisiiphilus*. Since no band was detected by the other genus, including *Pectinatus frisingensis*, in the present invention, it was certified that *Pectinatus cerevisiiphilus* can be specifically detected, and it can be identified.

### (2) Detection method of Pectinatus frisingensis

### Preparation of samples

Ten strains of three kinds of bacterial species of *Pectinatus* as shown in Table 1 were used in the method as described above. To confirm the specificity of primers for *Pectinatus frisingensis*, other bacteria and yeasts to be detected in the inspection of beer microorganisms, and the *E. coli* used often in laboratories, were used. After cultivating these strains at suitable cultures for amplification, the strains were collected by centrifugation. Then, the DNA from the strains was extracted in accordance with the description of SHIN-SEIKAGAKU-JIKKEN-KOZA 2, Nucleic acid I, Separation and Purification, page 20-21 (edited by Japan Biochemical Learned Society, Tokyo-Kagaku-Dojin) to obtain each one ml of DNA solution.

### Synthesis of Primers

From the base sequences of 16S ribosomal RNA gene of *Pectinatus* (KARL HEINZ SCHLEIFER, and HELGA SEIDEL-RUFER *et al*, Int. J. Syst. Bacteriol. 40(1): 19-27, 1990), the following four sequences were selected, and oligonucleotides having these sequences were chemically synthesized.

### PCR

To a sterilized tube of 0.5 ml, the above sample solution of 1 µl, sterilized water of 78.5 µl, 10 x buffer for reaction of 10 µl (manufactured by Toyobo Co., Ltd., 10 x TAP), 6 µl of water solution of 25 mM magnesium chloride), one µl of 20 mM dNTPs, each one µl of 100 µM of primer SEQ ID NO.10 and 100 µM of primer SEQ ID NO.12 (or Primers SEQ ID NOS.11 and 13, or SEQ ID NOS. 10 and 13), and 0.5 µl of 5U/ µl Taq DNA polymerase (manufactured by Toyobo Co., Ltd., TAP-101) were added, and 100 µl of reaction solution was prepared. PCR was repeated for 30 cycles, each cycle having the following temperature conditions:
thermal denaturing; 94°C for one minute,
annealing; 54°C for one minute and
elongation; 74°C for one minute.

Before the cycle reaction, the solution was reacted at a temperature of 98°C for one minute. After the conclusion of the reaction, the solution was heated to 74°C for three minutes. The program tempcontrol system PC-800 manufactured by ASTEC company was used as a thermal control unit.

### Detection

Using 10 µl of reaction solution after the PCR, electrophoresis was conducted by 3.5% (w/v) agarose gel at 100V constant voltage for 30 minutes. At the same time, ø X174/HincII was electrophoresed as a molecular weight marker. After the electrophoresis, the gel was dyed in a ethidium bromide solution (about 0.5 µg/ml) for 15 minutes, the gel was observed under ultraviolet irradiation, and photographed. By the observation or photograph of the gel, the base length of the amplified products was determined from relative migration distance to the molecular weight marker.

### Result

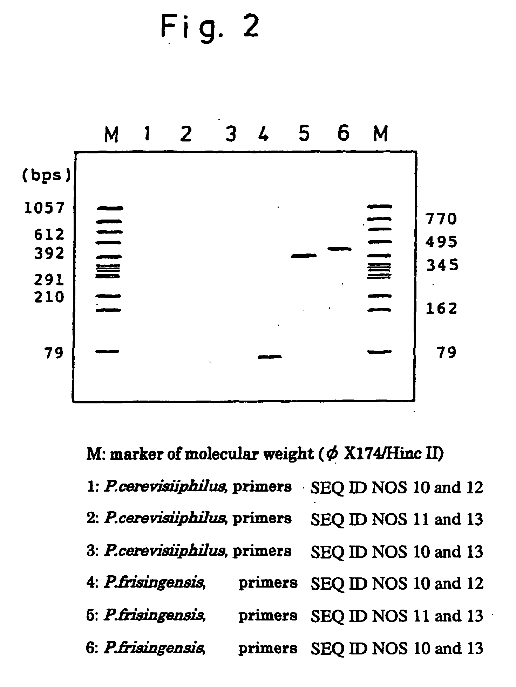

The result of the electrophoresis of the samples of genus numbers 2 and 5 in Table 1 after PCR is shown in Fig. 2.

The result of detected bands of all tested genus is shown in Table 3.

**Table 3**

| Genus No. | Combination of primers | | |
|---|---|---|---|
| | SEQ ID NOS. 10 & 12 | SEQ ID NOS.11 & 13 | SEQ ID NOS.10 & 13 |
| 1 | - | - | - |
| 2-4 | - | - | - |
| 5 | 0.07 | 0.39 | 0.44 |
| 6 | 0.07 | 0.39 | 0.44 |
| 7 | 0.07 | 0.39 | 0.44 |
| 8 | 0.07 | 0.39 | 0.44 |
| 9 | 0.07 | 0.39 | 0.44 |
| 10-26 | - | - | - |

In the table, figures show base length (kilo base pairs) of detected bands, and (-) shows no band.

From the result, when each oligonucleotide of SEQ ID NOS.10-13 was used as a primer of the PCR in the combinations shown in Table 3, a band was detected only by *Pectinatus frisingensis*, and the band showed a base length equal to that of the target sequence. Therefore, it is shown that each oligonucleotide properly recognizes the sequence targeted by the 16S ribosomal RNA gene of *Pectinatus frisingensis*. Since no band was detected by the other genus, including *Pectinatus cerevisiiphilus,* in the present invention, it was certified that *Pectinatus frisingensis* can be specifically detected, and it can be identified.

### (3) Detection method of Pectinatus sp. DSM20764

### Preparation of samples

In the *Pectinatus, Pectinatus cerevisiiphilus* (DSM20467), *Pectinatus frisingensis* (DSM6306) and *Pectinatus* sp. DSM20764 were used. To confirm the specificity of the primers for *Pectinatus* sp. DSM20764, other obligate anaerobic bacteria as shown in Table 4 were used. After cultivating these bacteria at suitable cultures for amplification, the strains were collected by centrifugation. Then, the DNA from the strains were extracted in accordance with the description of SHIN-SEIKAGAKU-JIKKEN-KOZA 2, Nucleic acid I, Separation and Purification, page 20-21 (edited by Japan Biochemical Learned Society, Tokyo-Kagaku-Dojin) to obtain DNA solution.

**Table 4**

| Bacteria No. | Bacteria type | Name of strain | Remarks |
|---|---|---|---|
| 1 | *Pectinatus cerevisiiphilus* | DSM20467 | type strain |
| 2 | *Pectinatus frisingensis* | DSM6036 | type strain |
| 3 | *Pectinatus* sp. | DSM20764 | |
| 4 | *Megasphaera cerevisiae* | DSM20462 | type strain |
| 5 | *Megasphaera cerevisiae* | JCM6129 | |
| 6 | *Megasphaera elsdenii* | JCM1772 | type strain |
| 7 | *Selenomonas lacticifex* | DSM20757 | type strain |
| 8 | *Zymophilus paincivorans* | DSM20756 | type strain |
| 9 | *Zymophilus raffinosivorans* | DSM20765 | type strain |

### Determination of the base sequence of the 16S ribosomal RNA gene of Pectinatus sp. DSM20764

Using DNA solution of the *Pectinatus* sp. DSM20764 prepared as described above as a template, PCR was conducted by the primer attached a M13 primer sequence to the 5' side of a universal primer in common with many bacteria, which were described in Modem Microbiological Methods 'Nucleic Acid Techniques in Bacterial Systematics' "16S/23S rRNA Sequencing" p115-175 (J. Wiley & Sons Ltd., New York).

After the PCR, amplified DNA fragments were separated and cut by agarose electrophoresis, eluted from the gel using Trade name SUPREC™-01 (manufactured by Takara Shuzo), and recovered by ethanol precipitate. Using the resulting DNA fragments as a template, a sequencing reaction was conducted. Trade name IRD41 Infrared Dye Labeled M13 Forward primer (manufactured by Nisshinbo, and sold by Aroka Ltd., Co.) was used as a sequencing primer, and SequiTherm™ Long-Read™ Cycle Sequencing Kit-LC (manufactured by EPICENTRE TECHNOLOGIES Company) was used as reaction solution. The base sequence was determined by 4000L Long ReadIR™ DNA Sequencing System (manufactured by LI-COR company).

The resulting 16S ribosomal RNA gene sequence of the *Pectinatus* sp. DSM20764 is SEQ ID NO.1.

### Synthesis of primer

Oligonucleotides, one of which has the same sequence as that of bases No. 630 to No. 647 on the sequence of the 16S ribosomal RNA gene of the *Pectinatus* sp. DSM20764 in SEQ ID NO.1 and the other of which has the complementary sequence to that of bases No. 1004 to No. 1022, were chemically synthesized.

### Detection and identification of Pectinatus sp. DSM20764 using a primer

PCR of DNA solution of each bacterium obtained by preparation of samples was conducted. The PCR was repeated for 35 cycles, and each cycle had the following temperature conditions:
thermal denaturation; 94°C for 30 seconds,
annealing; 55°C for 30 seconds and
elongation; 72°C for 30 seconds.

After the conclusion of PCR, the solution was electrophoresed by agarose gel at 100V constant voltage for 30 minutes. At the same time, ø X174/HincII was electrophoresed as a molecular weight marker. After the electrophoresis, the gel was dyed in an ethidium bromide solution (about 0.5 µg/ml) for 15 minutes, the gel was observed under ultraviolet irradiation, and photographed. By the observation or photograph of the gel, the base length of the amplified products was determined from relative migration distance to the molecular weight marker.

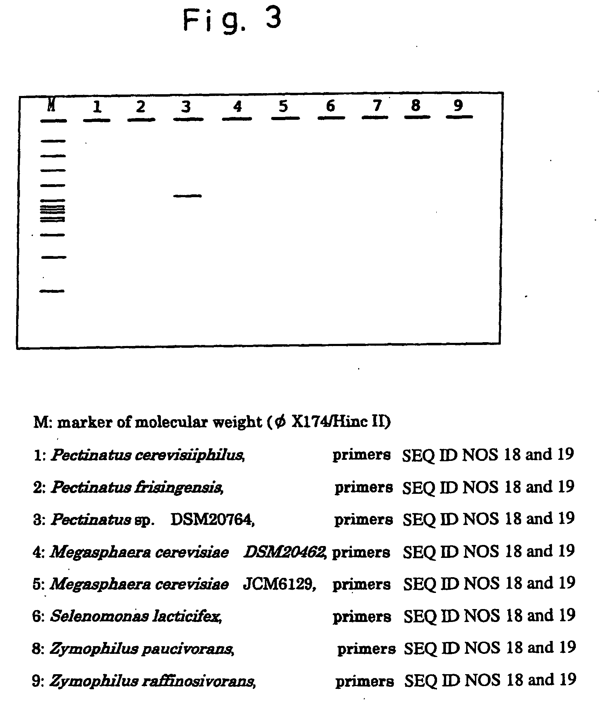

From the result, as shown in Fig. 3, a band of about 390 bps was detected only for *Pectinatus* sp. DSM20764. When the above oligonucleotides were used as a primer for the PCR, a band having the object length was detected only by *Pectinatus* sp. DSM20764. Therefore, it is shown that each oligonucleotide properly recognizes the sequence targeted by the 16S ribosomal RNA gene of *Pectinatus* sp. DSM20764. Since no band was detected by the same genus, including *Pectinatus cerevisiiphilus* and *Pectinatus frisingensis*, in the present invention, it was certified that *Pectinatus* sp. DSM20764 can be specifically detected, and it can be identified.

Conventionally it takes at least about ten days to identify the kind of *Pectinatus.* On the other hand, the present invention does not always need preculture and separation culture of the bacteria. Within one to three days, quickly and reliably, it is possible to detect and identify *Pectinatus cerevisiiphilus, Pectinatus frisingensis* and *Pectinatus* sp. DSM20764 in the sample. Further, since the PCR method can be easily conducted, a skilled operator is unnecessary. It is possible to detect in high reliability and low cost. By great progress of the PCR peripheral apparatus, it becomes possible to automatically detect the *Pectinatus* in the present invention.

## Claims

1. An oligonucleotide **characterized in that** the oligonucleotide targets a nucleotide sequence encoding a 16S ribosomal RNA gene of the genus *Pectinatus* to selectively detect *P. cerevisiiphilus* belonging to the genus *Pectinatus* in a test sample, wherein the oligonucleotide is complementary to the nucleotide sequence, and is one of the following: or one of the corresponding complementary sequences, (SEQ ID NOS. 6-9).

2. An oligonucleotide **characterized in that** the oligonucleotide targets a nucleotide sequence encoding a 16S ribosomal RNA gene of the genus *Pectinatus* to selectively detect *P. frisingensis* belonging to the genus *Pectinatus* in a test sample, wherein the oligonucleotide is complementary to the nucleotide sequence, and is one of the following: or one of the corresponding complementary sequences, (SEQ ID NOS. 14-17).

3. An oligonucleotide **characterised in that** the oligonucleotide targets a nucleotide sequence encoding a 16S ribosomal RNA gene of the genus *Pectinatus* to selectively detect *Pectinatus* sp. DSM20764 belonging to the genus *Pectinatus* in a test sample, wherein the oligonucleotide is complementary to the nucleotide sequence, and is one of the following: or one of the corresponding complementary sequences, (SEQ ID NOS. 20-21).

## Patentansprüche

1. Ein Oligonukleotid, **dadurch gekennzeichnet, dass** das Oligonukleotid-Ziel eine Nukleotidsequenz kodierend für ein 16S ribosomale RNA-Gen der Gattung *Pectinatus* ist, um selektiv *P. cerevisiiphilus* gehörend zur Gattung *Pectinatus* in einer Testprobe zu detektieren, worin das Oligonukleotid komplementär zu der Nukleotidsequenz ist und eines der folgenden ist: oder eines der korrespondierenden komplementären Sequenzen ist (SEQ ID NOS. 6 - 9).

2. Ein Oligonukleotid, **dadurch gekennzeichnet, dass** das Oligonukleotid-Ziel eine Nukleotidsequenz kodierend für ein 16S ribosomale RNA-Gen der Gattung *Pectinatus* ist, um selektiv *P. frisingensis* gehörend zur Gattung *Pectinatus* in einer Testprobe zu detektieren, worin das Oligonukleotid komplementär zu der Nukleotidsequenz ist und eines der folgenden ist: oder eines der korrespondierenden komplementären Sequenzen ist (SEQ ID NOS. 14 - 17).

3. Ein Oligonukleodid, **dadurch gekennzeichnet, dass** das Oligonukleotid-Ziel eine Nukleotidsequenz kodierend für ein 16S ribosomale RNA-Gen der Gattung *Pectinatus* ist, um selektiv *Pectinatus sp.* DSM20764 gehörend zur Gattung *Pectinatus* in einer Testprobe zu detektieren , worin das Oligonukleotid komplementär zu der Nukleotidsequenz ist und eines der folgenden ist: oder eines der korrespondierenden komplementären Sequenzen ist (SEQ ID NOS. 20 - 21).

## Revendications

1. Oligonucléotide **caractérisé en ce que** l'oligonucléotide vise une séquence de nucléotides codant pour un gène ARN ribosomique 16S du genre *Pectinatus* pour détecter sélectivement *P*. *cerevisiiphilus* appartenant au genre *Pectinatus* dans un échantillon d'essai, dans lequel l'oligonucléotide est complémentaire de la séquence de nucléotides, et est l'un des suivants : ou l'une des séquences complémentaires correspondantes,(SEQ ID NOS 6-9).

2. Oligonucléotide **caractérisé en ce que** l'oligonucléotide vise une séquence de nucléotides codant pour un gène ARN ribosomique 16S du genre *Pectinatus* pour détecter sélectivement *P. frisingensis* appartenant au genre *Pectinatus* dans un échantillon d'essai, dans lequel l'oligonucléotide est complémentaire de la séquence de nucléotides, et est l'un des suivants : ou l'une des séquences complémentaires correspondantes, (SEQ ID NOS 14-17).

3. Oligonucléotide **caractérisé en ce que** l'oligonucléotide vise une séquence de nucléotides codant pour un gène ARN ribosomique 16S du genre *Pectinatus* pour détecter sélectivement *Pectinatus* sp. DSM20764 appartenant au genre *Pectinatus* dans un échantillon d'essai, dans lequel l'oligonucléotide est complémentaire de la séquence de nucléotides, et est l'un des suivants : ou l'une des séquences complémentaires correspondantes, (SEQ ID NOS 20-21).


### SEQUENCE LISTING

<110> ASAHI Breweries, LTD.
<120> Detection of bacterium belonging to the genus Pectinatus
<130> P20406EP
<140> EP 96939308.1
   <141> 1996-11-27
<150> PCT/JP96/03464
   <151> 1996-11-27
<150> JP 331172/95
   <151> 1995-11-28
<150> JP 331173/95
   <151> 1995-11-28
<160> 21
<170> PatentIn Ver. 2.1
<210> 1
   <211> 1542
   <212> DNA
   <213> Pectinatus sp.
<400> 1
   agagtttgat cctggctcag gacgaacgct ggcggcgtgc ttaacacatg caagtcgaac 60
   gggactttta tttcggtaaa agtctagtgg caaacgggtg agtaacgcgt aggcaaccta 120
   ccttcaagat ggggacaaca tcccgaaagg ggtgctaata ccgaatgttg taagagtact 180
   gcatggtact tttaccaaag gcggctttta gctgttactt ggagatgggc ctgcgtctga 240
   ttagctagtt ggtgacggta atggcgcacc aaggcaacga tcagtagccg gtctgagagg 300
   atggacggcc acattgggac tgagacacgg cccagactcc tacgggaggc agcagtgggg 360
   aatcttccgc aatgggcgaa agcctgacgg agcaacgccg cgtgaacgag gaaggtcttc 420
   ggatcgtaaa gttctgttgc aggggacgaa tggcattagt gctaatacca ctaatgaatg 480
   acggtaccct gttagaaagc cacggctaac tacgtgccag cagccgcggt aatacgtagg 540
   cggcaagcgt tgtccggaat cattgggcgt aaagggagcg caggcggaca tttaagcgga 600
   tcttaaaagt gcggggctca accccgtgat ggggtccgaa ctgaatgtct tgagtgcagg 660
   agaggaaagc ggaattccca gtgtagcggt gaaatgcgta gatattggga agaacaccag 720
   tggcgaaggc ggctttctgg actgtaactg acgctgaggc tcgaaagcca gggtagcgaa 780
   cgggattaga taccccggta gtcctggccg taaacgatgg atactaggtg tagggggtat 840
   cgaccccccc tgtgccggag ttaacgcaat aagtatcccg cctggggagt acggccgcaa 900
   ggctgaaact caaaggaatt gacgggggcc cgcacaagcg gtggagtatg tggtttaatt 960
   cgacgcaacg cgaagaacct taccagggct tgacattgat tgacgcattc agagatggat 1020
   gcttcctctt cggaggacaa gaaaacaggt ggtgcatggc tgtcgtcagc tcgtgtcgtg 1080
   agatgttggg ttaagtcccg caacgagcgc aacccctatc atttgttgcc agcacgtaac 1140
   ggtgggaact caaatgagac tgccgcggac aacgcggagg aaggcgggga tgacgtcaag 1200
   tcatcatgcc ccttacgtcc tgggctacac acgtactaca atgggataca cagagggaag 1260
   caaaggagcg atccggagcg gaacccaaaa aatatccccc agttcggatt gcaggctgca 1320
   actcgcctgc atgaagtcgg aatcgctagt aatcgcaggt cagcatactg cggtgaatac 1380
   gttcccgggc cttgtacaca ccgcccgtca caccacgaaa gtcattcaca cccgaagccg 1440
   gctaagggcc ttatggaacc gaccgtctaa ggtgggggcg atgattgggg tgaagtcgta 1500
   acaaggtagc cgtatcggaa ggtgcggctg gatcacctcc tt                    1542
<210> 2
   <211> 18
   <212> DNA
   <213> Artificial Sequence
<220>
   <223> Description of Artificial Sequence: Oligonucleotide
<400> 2
   caggcggatg actaagcg 18
<210> 3
   <211> 18
   <212> DNA
   <213> Artificial Sequence
<220>
   <223> Description of Artificial Sequence: Oligonucleotide
<400> 3
   tgggattcga actggtca 18
<210> 4
   <211> 18
   <212> DNA
   <213> Artificial Sequence
<220>
   <223> Description of Artificial Sequence: Oligonucleotide
<400> 4
   ctcaagatga ccagttcg 18
<210> 5
   <211> 20
   <212> DNA
   <213> Artificial Sequence
<220>
   <223> Description of Artificial Sequence: Oligonucleotide
<400> 5
   aatatgcatc tctgcatacg 20
<210> 6
   <211> 18
   <212> DNA
   <213> Artificial Sequence
<220>
   <223> Description of Artificial Sequence: Oligonucleotide
<400> 6
   cgcttagtca tccgcctg 18
<210> 7
   <211> 18
   <212> DNA
   <213> Artificial Sequence
<220>
   <223> Description of Artificial Sequence: Oligonucleotide
<400> 7
   tgaccagttc gaatccca 18
<210> 8
   <211> 18
   <212> DNA
   <213> Artificial Sequence
<220>
   <223> Description of Artificial Sequence: Oligonucleotide
<400> 8
   cgaactggtc atcttgag 18
   <210> 9
   <211> 20
   <212> DNA
   <213> Artificial Sequence
<220>
   <223> Description of Artificial Sequence: Oligonucleotide
<400> 9
   cgtatgcaga gatgcatatt 20
<210> 10
   <211> 18
   <212> DNA
   <213> Artificial Sequence
<220>
   <223> Description of Artificial Sequence: Oligonucleotide
<400> 10
   caggcggaac attaagcg 18
<210> 11
   <211> 18
   <212> DNA
   <213> Artificial Sequence
<220>
   <223> Description of Artificial Sequence: Oligonucleotide
<400> 11
   atggggtccg aactgagg 18
   <210> 12
   <211> 18
   <212> DNA
   <213> Artificial Sequence
<220>
   <223> Description of Artificial Sequence: Oligonucleotide
<400> 12
   ctcaagaacc tcagttcg 18
<210> 13
   <211> 20
   <212> DNA
   <213> Artificial Sequence
<220>
   <223> Description of Artificial Sequence: Oligonucleotide
<400> 13
   aatatccatc tctggatacg 20
<210> 14
   <211> 18
   <212> DNA
   <213> Artificial Sequence
<220>
   <223> Description of Artificial Sequence: Oligonucleotide
<400> 14
   cgcttaatgt tccgcctg 18
   <210> 15
   <211> 18
   <212> DNA
   <213> Artificial Sequence
<220>
   <223> Description of Artificial Sequence: Oligonucleotide
<400> 15
   cctcagttcg gaccccat 18
<210> 16
   <211> 18
   <212> DNA
   <213> Artificial Sequence
<220>
   <223> Description of Artificial Sequence: Oligonucleotide
<400> 16
   cgaactgagg ttcttgag 18
<210> 17
   <211> 20
   <212> DNA
   <213> Artificial Sequence
<220>
   <223> Description of Artificial Sequence: Oligonucleotide
<400> 17
   cgtatccaga gatggatatt 20
<210> 18
   <211> 18
   <212> DNA
   <213> Artificial Sequence
<220>
   <223> Description of Artificial Sequence: Oligonucleotide
<400> 18
   tggggtccga actgaatg 18
<210> 19
   <211> 19
   <212> DNA
   <213> Artificial Sequence
<220>
   <223> Description of Artificial Sequence: Oligonucleotide
<400> 19
   gcatccatct ctgaatgcg 19
<210> 20
   <211> 18
   <212> DNA
   <213> Artificial Sequence
<220>
   <223> Description of Artificial Sequence: Oligonucleotide
<400> 20
   cattcagttc ggacccca 18
<210> 21
   <211> 19
   <212> DNA
   <213> Artificial Sequence
<220>
   <223> Description of Artificial Sequence: Oligonucleotide
<400> 21
   cgcattcaga gatggatgc                                                    19